## Europäisches Patentamt
## European Patent Office
## Office européen des brevets

(19)

(11) Veröffentlichungsnummer: **0 088 951**
**B1**

(12) **EUROPÄISCHE PATENTSCHRIFT**

(45) Veröffentlichungstag der Patentschrift:
**24.06.87**

(51) Int. Cl.⁴: **A 61 K 9/32**

(21) Anmeldenummer: **83102056.5**

(22) Anmeldetag: **03.03.83**

(54) **Verfahren zum Überziehen von Arzneiformen mittels eines in Wasser dispergierten Überzugsmittels.**

(30) Priorität: **11.03.82 DE 3208791**

(43) Veröffentlichungstag der Anmeldung:
**21.09.83 Patentblatt 83/38**

(45) Bekanntmachung des Hinweises auf die Patenterteilung:
**24.06.87 Patentblatt 87/26**

(84) Benannte Vertragsstaaten:
**AT BE CH DE FR GB IT LI NL SE**

(56) Entgegenhaltungen:
**EP - A - 0 050 191**
**DE - A - 2 512 238**
**DE - A - 3 049 179**
**FR - A - 2 145 642**

(73) Patentinhaber: **Röhm GmbH, Kirschenallee
Postfach 4242, D-6100 Darmstadt 1 (DE)**

(72) Erfinder: **Lehmann, Klaus, Dr., Schillerstrasse 85,
D-6101 Rossdorf 1 (DE)**
Erfinder: **Dreher, Dieter, De-la-Fosse-Weg 35,
D-6100 Darmstadt (DE)**
Erfinder: **Rauch, Hubert, Odenwaldstrasse 6,
D-6108 Weiterstadt 1 (DE)**
Erfinder: **Siol, Werner, Dr., Mühlbergstrasse 4,
D-6102 Pfungstadt (DE)**

## Beschreibung

### Gebiet der Erfindung

Die Erfindung betrifft ein Verfahren zum Überziehen von Arzneiformen mittels eines in Wasser dispergierten Überzugsmittels.

### Stand der Technik

Aus DE-C-21 35 073 ist es bekannt, Arzneiformen mittels einer wässrigen Dispersion eines aus Vinylmonomeren gebildeten Kunststoffes, der zu 10 bis 55 Gew.-% aus Monomeren mit einer Carboxyl- oder Aminoestergruppe aufgebaut ist, zu überziehen. Die Beschichtung mit wässrigen Überzugsmitteldispersionen hat vor der Anwendung organischer Überzugsmittellösungen den Vorteil, dass die mit den organischen Lösungsmitteln verbundene Feuergefahr und Umweltbelastung vermieden wird, aber den Nachteil, dass die Herstellung der wässrigen Überzugsmitteldispersionen schwierig und von dem Arzneimittelhersteller nicht zu bewerkstelligen ist. Er muss daher das Bindemittel zusammen mit dem dispergierenden Wasser beziehen und lagern. Dagegen kann er organische Überzugsmittellösungen aus dem pulverförmigen Überzugsmittel ohne Schwierigkeiten selbst herstellen.

Nach DE-C-25 12 238 lassen sich zwar wässrige Dispersionen von Bindemitteln für Arzneimittelüberzüge durch Sprühtrocknen in ein Pulver überführen, jedoch muss dieses zur Anwendung in einem organischen Lösungsmittel gelöst werden.

Nach der DE-A-30 49 179 erzeugt man Arzneimittelüberzüge aus einem sprühgetrockneten Emulsionspolymerisat-Pulver, indem man es in der wässrigen Lösung eines nicht flüchtigen Weichmachungsmittels suspendiert und die Suspension auf Arzneiformen aufträgt und erwärmt, wodurch ein Teil des Wassers verdampft und das suspendierte Überzugsmittelpulver in dem Weichmacher in Lösung geht. Diese Lösung fliesst zu einer Überzugsschicht zusammen und erstarrt beim Erkalten. Dieses Verfahren wird als «Thermogelierung» bezeichnet. Der Filmbildungsvorgang unterscheidet sich wesentlich von dem echter wässriger Latizes. Dort ist der Kohäsionsdruck zwischen den Latexteilchen beim allmählichen Verdunsten des Wassers entscheidend, d.h. die Latexteilchen gehen unmittelbar als solche in den zusammenhängenden Film über und nicht über die Zwischenstufe einer Lösung.

Das Thermogelierungsverfahren erfüllt nicht immer alle Forderungen, die an ein Überzugsverfahren für Arzneiformen und an die dafür eingesetzten Überzugsmittel gestellt werden. Da das Überzugsmittelpulver nicht als Latex redispergiert, sondern in grösseren Partikeln suspendiert wird, ist die entstehende Suspension oft nicht über ausreichend lange Zeit lagerfähig. Um einen glatten und vollkommen porenfreien Überzug zu erhalten, müssen erhebliche Mengen an Weichmachern und hohe Gelierungstemperaturen angewendet werden; andernfalls entstehen rauhe, mehr oder weniger poröse Überzüge oder es müssen sehr dicke Schichten aufgetragen werden.

### Aufgabe und Lösung

Die Erfinder haben sich die Aufgabe gestellt, die Vorteile der Arzneiformbeschichtung mit einer Überzugsmitteldispersion mit denen des Einsatzes von trockenen Überzugsmittelpulvern zu verbinden. Es war ein Verfahren zu entwickeln, das keine grossen Weichmachermengen und keine hohen Filmbildungstemperaturen erfordert, aber glatte und porenfreie Überzüge bildet. Weiterhin soll ein längere Zeit lagerfähiges wässriges Überzugsmittel verwendet werden. Diese Vorteile werden durch das Verfahren zum Überziehen von Arzneiformen mittels eines wässrigen Überzugsmittels, welches als Bindemittel einen Latex eines Emulsionspolymerisats aus

A) 20 bis 85 Gew.-% Einheiten wenigstens eines Alkylesters der Acryl- und/oder Methacrylsäure,

B) 80 bis 15 Gew.-% Einheiten wenigstens eines zur Salzbildung befähigten, radikalisch polymerisierbaren Monomeren,

C) gegebenenfalls bis zu 30 Gew.-% Einheiten von einem oder mehreren anderen radikalisch polymerisierbaren Monomeren

enthält, durch Auftragen des wässrigen Überzugsmittels auf die Arzneiformen und Verdunsten des Wassers unter Bedingungen, unter denen die Latexteilchen verfilmen, erreicht, das dadurch gekennzeichnet ist, dass man das Emulsionspolymerisat, worin bis zu 10 Gew.-% (bezogen auf das Emulsionspolymerisat) der Monomereinheiten B in der Salzform vorliegen können, in Form eines trockenen Pulvers, dessen Körnchen aus lose aggregierten Feinpartikeln aufgebaut sind, in Wasser einträgt und in Gegenwart einer Menge eines zur Salzbildung mit den Monomereinheiten B befähigten Mittels, die so bemessen ist, dass nach ihrer Umsetzung mit den Monomereinheiten B insgesamt 0,1 bis 10, vorzugsweise 0,5 bis 5 Gew.-% dieser Einheiten, bezogen auf das Gewicht des Emulsionspolymerisats, in Salzform vorliegen, zu wenigstens 80 Gew.-% zu Latexteilchen redispergiert.

Als Bindemittel wird die filmbildende Komponente des Überzugsmittels bezeichnet, unabhängig davon, ob sie gemeinsam mit ungelösten Hilfsstoffen angewendet wird. Dementsprechend kann die Filmbildung entweder in Abwesenheit von Zusätzen zu einem klaren Polymerisatüberzug führen oder in Anwesenheit von ungelösten Zusätzen zu einem deckenden Überzug. Die Filmbildung tritt unmittelbar aus dem Latexzustand ein. In dem eingesetzten pulverförmigen Emulsionspolymerisat sind die Latexteilchen in ihrer ursprünglichen Gestalt vorhanden, aber zu lockeren Aggregaten zusammengeballt. Sie können daher nahezu vollständig zu einzeln dispergierten Latexteilchen verteilt werden.

### Vorteile der Erfindung

Das Bindemittel zur Bereitung eines wässrigen Überzugsmittels kann bis zur Herstellung des Überzugsmittels raum- und gewichtssparend und ohne Gefahr eines Qualitätsverlustes als trockenes Pulver gelagert werden. Bei der Redispergierung können zusammen mit dem Bindemittelpulver alle übrigen Zusätze in das Überzugsmittel eingearbeitet werden. Man erhält ein wässriges Überzugsmittel, das über längere Zeiträume lagerfähig ist.

Obwohl das Überzugsmittel zur Einstellung der gewünschten Filmhärte und -elastizität auch übliche Weichmachungsmittel enthalten kann, werden davon keine Mengen benötigt, die den Film während der Herstellung weich und klebrig machen. Dies ist ein wesentlicher Vorzug des erfindungsgemässen Verfahrens vor der Herstellung von Überzügen aus organischen Lösungen, die entweder als solche eingesetzt oder durch das Verfahren der Thermogelierung intermediär gebildet werden. Dabei wird stets ein stark klebriges Stadium während des Trocknungsprozesses durchlaufen.

Die verminderte, in der Regel sogar völlig fehlende Klebrigkeit ist auch eine Folge der verminderten Filmbildungstemperatur. Die Filmbildung aus Latexdispersionen tritt deutlich unterhalb des Temperaturbereichs ein, in dem das Überzugsmittel klebrig wird, so dass dieser Temperaturbereich ohne weiteres vermieden werden kann.

Anwendungsgebiete

Je nach der sauren oder basischen Natur der salzbildenden Gruppen in dem Emulsionspolymerisat entstehen magensaftresistente und darmsaftlösliche oder magensaftlösliche Überzüge. Es ist überraschend, dass die teilneutralisierten, Carboxylgruppen enthaltenden Überzüge im sauren und sogar im neutralen Milieu ungelöst bleiben. Bei niedrigem Gehalt an Carboxylgruppen lösen sich die Überzüge im schwach alkalischen Bereich langsam und mit steigendem Carboxylgehalt schneller auf. Ort und Zeit der Auflösung lassen sich daher den galenischen Anforderungen entsprechend einstellen.

Die Überzugsmittel können zur Herstellung einschichtiger Umhüllungen oder als eine von mehreren Umhüllungen auf Arzneiformen aufgebracht werden. Ebenso wie die bekannten Überzugsmittel mit einem Gehalt organisch gelöster Bindemittel von gleicher oder ähnlicher chemischer Zusammensetzung können auch die erfindungsgemäss erzeugten Überzüge Grund- oder Decküberzüge oder Zwischenschichten mehrschichtiger Überzüge bilden. Als Arzneiformen, die erfindungsgemäss beschichtet werden können, seien Tabletten, Dragees, Kapseln, Pellets und Granulate genannt, wobei die letzteren auch zu Gerüsttabletten verpresst werden können.

Das Emulsionspolymerisat

kann nach dem in DE-C-21 35 073 beschriebenen Verfahren hergestellt worden sein. Emulsionspolymerisate mit einem Gehalt an carboxylgruppenhaltigen Monomeren bis zu etwa 70 Gew.-% sind z.B. nach dem Verfahren der EP-A-0 073 296 zugänglich.

Als Alkylester der Acryl- oder Methacrylsäure (A) sind am Aufbau des Emulsionspolymerisats vor allem diejenigen mit 1 bis 8 C-Atomen im Alkylrest beteiligt. Bevorzugt sind Methylacrylat und -methacrylat sowie Äthylacrylat und -methacrylat.

Die zur Salzbildung befähigte Monomerkomponente (B) kann Carboxylgruppen enthalten und ist dann mit Basen zur Salzbildung befähigt, oder sie kann primäre, sekundäre oder tertiäre Aminogruppen enthalten und ist dann mit Säuren zur Salzbildung befähigt. Geeignete Säuremonomere sind vorzugsweise Acrylsäure oder Methacrylsäure oder deren Gemische. Andere Säuremonomere sind z.B. Malein-, Fumar- und Itakonsäure sowie Halbester oder Halbamide dieser Säuren. Geeignete Monomere mit Aminogruppen sind Vinylimidazol, Vinylimidazolin, Vinylimidazolidin, Vinylpyridin, Monoalkyl- bzw. Dialkylaminoalkylester oder Monoalkyl oder Dialkylaminoalkylamide von ungesättigten polymerisierbaren Carbonsäuren, wie z.B. Cyclohexylaminoäthylacrylat und -methacrylat, Dimethylaminoäthylacrylat und -methacrylat, Diäthylaminoäthylacrylat und -methacrylat, Morpholinoäthylacrylat und -methacrylat, Piperidinoäthylacrylat und -methacrylat, 4-Dimethylamino-butylacrylat und -methacrylat, Dimethylaminoneopentylacrylat und -methacrylat und N-Dimethylaminoäthylacrylamid und -methacrylamid.

Daneben können gegebenenfalls weitere Comonomere (C) am Aufbau des Polymerisats beteiligt sein, wie z.B. Acryl- oder Methacrylamid, Hydroxyalkylester der Acryl- oder Methacrylsäure, Vinylester, Vinylpyrrolidon oder niedere Olefine, wie Äthylen oder Propylen, oder Styrol.

Das Mengenverhältnis der Monomerenkomponenten (A), (B) und gegebenenfalls (C) richtet sich einerseits nach den Erfordernissen des Überzugsverfahrens, andererseits nach dem galenischen Verhalten des Überzugs. Das Emulsionspolymerisat muss bei Raumtemperatur in Pulverform im unverglasten Zustand lagerfähig sein. Das setzt eine Glastemperatur oberhalb 25 °C, vorzugsweise oberhalb 40 °C voraus. Die Glastemperatur wird durch die Komponenten A, ausser Methyl- und Äthylmethacrylat, gesenkt und durch die letztgenannten Ester und die Mehrzahl der Monomeren der Komponente B erhöht. Mit der Senkung der Glastemperatur geht eine Zunahme der Filmelastizität und Dehnbarkeit einher. Umgekehrt steigt mit der Glastemperatur die Härte und Sprödigkeit des Films.

Der Anteil der weichmachenden Monomeren, wie Acrylsäurealkylester und höhere Methacrylsäurealkylester, muss daher hoch genug sein, um die Filmbildung bei mässiger Temperatur zu ermöglichen und um einen Film von ausreichender Elastizität zu ergeben. Die hartmachenden Monomeren, mit denen die Glastemperatur auf die gewünschte Höhe gebracht werden kann, können den Komponenten A oder/und B angehören. Unter den zu B gehörenden Monomeren wirken vor allem Acryl- und Methacrylsäure und deren Salze hartmachend, die Aminoalkylamide in geringerem Masse und Aminoalkylester noch weniger. Wenn daher mit den Monomeren B allein die notwendige Härte nicht erreicht wird, wird Methyl-oder Äthylmethacrylat als Teil der Komponente A hinzugenommen.

Magensaftresistente Überzüge werden erhalten, wenn die Monomeren der Komponente B Carboxyl- bzw. Carboxylatgruppen enthalten. Bei hohen Gehalten an diesen Gruppen sind die Überzüge schnell im alkalischen Darmsaft löslich, bei geringeren Gehalten lösen sie sich langsamer oder werden durch Quellung diffusionsdurchlässig. Aminogruppenhaltige Monomere machen den Überzug bei hohen Anteilen magensaftlöslich, bei niedrigen Anteilen in Magensaft quellbar und diffusionsdurchlässig.

Das Emulsionspolymerisat wird in der Regel in Gegenwart von anionischen, kationischen oder nichtionischen Emulgatoren oder deren verträglichen Gemischen in Form eines wässrigen Latex mit einem Feststoffgehalt von z.B. 30 bis 70 Gew.-% hergestellt. Bei der Gewinnung des trokkenen Polymerisatpulvers aus dem Latex ist darauf zu achten, dass die Latexteilchen als solche erhalten bleiben und nicht zu unzertrennbaren Aggregaten verkleben. Das gelingt, wenn bei der Isolierung des Polymerisats Temperaturen oberhalb der Mindestfilmbildungstemperatur vermieden werden. Geeignet sind vor allem die Verfahren der Sprühtrocknung und der Gefriertrocknung. Das letztgenannte Verfahren ist auch bei Polymerisaten an der unteren Grenze des Bereichs geeigneter Glastemperaturen noch anwendbar. Das getrocknete Emulsionspolymerisat liegt in Form eines feinen weissen Pulvers vor, dessen Körner aber in der Regel nicht aus einzelnen Latexteilchen, sondern aus lockeren Aggregaten vieler Latexteilchen bestehen, die sich ohne Kraftaufwand zerteilen lassen. Wenn beim Druck mit einer Nadel auf ein Pulverkörnchen splitterige Bruchstücke abspringen, so ist dies ein Zeichen dafür, dass die Latexteilchen verglast und unlösbar miteinander verbunden sind. Die Herstellung des pulverförmigen Emulsionspolymerisats ist nicht Gegenstand der Erfindung.

Das salzbildende Mittel

Das Emulsionspolymerisat enthält als salzbildende Komponente B Monomereinheiten mit sauren oder basischen Gruppen. Die Salzbildung der sauren Gruppen tritt durch Umsetzung mit einer Base ein. Als Basen kommen Alkalien, wie z.B. Natronlauge, Kalilauge, Soda, Pottasche, Natriumbikarbonat, Trinatriumphosphat, Trinatriumcitrat oder Ammoniak oder physiologisch verträgliche Amine, wie Triäthanolamin oder Tris-(hydroxymethyl)-aminomethan, in Betracht. Ammoniak ist besonders wirksam und schon in einer Konzentration von 0,1 Gew.-%, insbesondere 0,5 Gew.-%, redispergierwirksam, während andere Basen oft erst ab einer Konzentration von 1 Gew.-% eine ausreichende Wirksamkeit entfalten. Die aminogruppenhaltigen Monomereinheiten werden mit Säuren in die Salzform übergeführt. Als physiologisch verträgliche Säuren können z.B. Salzsäure, Phosphorsäure, Citronensäure, Essigsäure oder Weinsäure verwendet werden. Die Überführung eines begrenzten Anteils der salzbildenden Einheiten B in die Salzform ist für die Redispergierung des Emulsionspolymerisats von wesentlicher Bedeutung. Entscheidend ist nicht die Absolutmenge des salzbildenden Mittels, sondern die Gewichtsmenge an Einheiten B, die in die Salzform übergeführt werden. Wenn diese Menge unter 0,1 Gew.-%, bezogen auf das Gesamtgewicht des nicht neutralisierten Emulsionspolymerisats, liegt, so wird keine ausreichende Redispergierung erreicht. Wenn die Menge über 10 Gew.-% liegt, so gehen die Latexteilchen in einen stark gequollenen Zustand über oder sie lösen sich vollständig auf. In beiden Fällen kann kein befriedigender Film mehr erzeugt werden. Die Menge des salzbildenden Mittels wird vorzugsweise nicht grösser gewählt, als zur Überführung in eine hinreichend stabile Redispersion gerade ausreichend ist. Die günstigsten Ergebnisse werden im allgemeinen bei einem Anteil von 0,5 bis 5 Gew.-% salzförmiger Monomereinheiten B erhalten. Besonders bevorzugt ist der Bereich von 1,0 bis 3 Gew.-%.

Die Überführung der salzbildenden Gruppen in die Salzform kann in verschiedenen Stadien geschehen. Man kann das salzbildende Mittel oder einen Teil desselben z.B. schon der ursprünglichen wässrigen Dispersion zusetzen, aus der das Polymerisatpulver erzeugt wird. Wenn auch in der Regel das trockene Polymerisatpulver noch keine salzförmigen Gruppen enthält, können gewünschtenfalls bis zu 10 Gew.-% (bezogen auf das Emulsionspolymerisat) der Monomereinheiten B in der Salzform vorliegen. Das salzbildende Mittel kann dann, gegebenenfalls auch teilweise, in Form eines Pulvers mit dem Polymerisatpulver vermischt werden. Geeignete pulverförmige salzbildende Mittel sind z.B. Soda oder Citronensäure. Vorzugsweise wird das salzbildende Mittel erst beim Redispergieren eingesetzt. Man rührt z.B. das Polymerisatpulver in die wässrige Lösung des salzbildenden Mittels ein. Vorzugsweise wird es erst nach dem Einrühren des Polymerisatpulvers in Wasser zugegeben, zweckmässig in Form einer wässrigen Lösung. Das Polymerisatpulver geht dann in den Latexzustand über. Dies geschieht am besten unter mässigem Rühren. Wenn zu stark gerührt wird, kann Koagulat entstehen. Der Anteil an Koagulat oder von Aggregaten, die deutlich grösser als die ursprünglichen Latexteilchen sind, soll 20 Gew.-% des eingesetzten Polymerisatpulvers nicht überschreiten. Wenigstens 80 Gew.-% des Polymerisats sollen in Form von Latexteilchen mit Durchmessern von 0,1 bis 5 μm vorliegen. In der Regel entspricht die Teilchengrösse im wesentlichen der des ursprünglichen Latex oder sie ist infolge einer leichten Quellung geringfügig grösser. Es ist im Regelfall nicht erforderlich, aber in manchen Fällen hilfreich, beim Redispergieren eine kleine Menge eines geeigneten oberflächenaktiven Mittels zuzusetzen.

Das Überzugsmittel

besteht im einfachsten Fall aus der Redispersion mit einem Gehalt des Emulsionspolymerisats von z.B. 10 bis 40, vorzugsweise 20 bis 35 Gew.-%. In der Regel enthält das Überzugsmittel weitere Hilfsstoffe, wie z.B. Pigmente, Talkum, Glättemittel, Netzmittel, Glanzmittel, Weichmacher usw., die üblicherweise getrennt in Wasser dispergiert und später zugemischt werden. Diese Hilfsstoffe können aber auch direkt in die Redispersion eingearbeitet werden. Der Bindemittelanteil liegt bei 10 bis 100, vorzugsweise zwischen 30 und 90 Gew.-%, bezogen auf Gesamtfeststoffe. Feststoffgehalt und Viskosität des Überzugsmittels richten sich nach dem Auftragsverfahren. Im allgemeinen beträgt die Viskosität etwa 5 bis 40 mPa.s. Es können alle gebräuchlichen Auftragsverfahren angewendet werden, z.B. die üblichen Kesseldragierung oder die Beschichtung von Arzneiformen in einer Wirbelschichtapparatur.

Man trägt das Überzugsmittel in der Regel in einer Menge von 0,5 bis 10 mg Feststoff je Quadratzentimeter der Arzneiformoberfläche auf und erhält Schichtdicken von 1 bis 100 μm. Das Überzugsmittel kann – wie bei anderen Überzugsverfahren – portionsweise in mehreren Schichten aufgetragen werden, zwischen denen jeweils getrocknet werden kann. Ebenso kann man das Überzugsmittel kontinuierlich auftragen und gleichzeitig mittels leicht erwärmter Luft trocknen. Es genügt im allgemeinen, an der Oberfläche der zu überziehenden Arzneiformen, unter 40 °C, meistens sogar unter 35 °C, vorzugsweise 25 bis 35 °C zu erzeugen, wobei je nach Verdunstungsgeschwindigkeit und Gerätekonstruktion Zulufttemperaturen von 40 bis 70 °C erforderlich sind.

Beispiel 1

Durch Emulsionspolymerisation von gleichen Gewichtsteilen Methacrylsäure und Acrylsäureäthylester wurde eine Dispersion mit 30% Trockengehalt hergestellt, wobei der mittlere Durchmesser der Latexteilchen 100 mm betrug. Diese Dispersion wurde gefriergetrocknet und der Trockenrückstand zu einem grobkörnigen Pulver zerstossen. Nun wurden 3,6 g NaOH in 1 l Wasser gelöst und 300 g des obigen Pulvers unter Rühren eingetragen. Diese NaOH-Menge reicht aus, um einen Anteil von 2,6 Gew.-% des Polymerisats in die Na-Salzform überzuführen. Nach weiterem Rühren entsteht innerhalb von 5 min eine milchige Dispersion, in der durch Grindometermessung kein Partikel über 10 μm mehr enthalten war. Eine Korngrössenbestimmung mit dem Nanosizer ergab einen mittleren Durchmesser der Latexteilchen von 120 nm. 1000 g der so erhaltenen Dispersion wurden auf 3 kg Tabletten aufgesprüht. Es entstand ein glatter, gleichmässiger Filmüberzug, der in künstl. Magensaft nach DAB resistent ist; die Tabletten zerfallen anschliessend in künstl. Darmsaft pH 7,5 innerhalb von 15 min.

Beispiel 2

Aus einer 40%igen wässrigen Dispersion aus 30% Methacrylsäure und 70% Methacrylsäuremethylester wurde durch Sprühtrocknung bei 60 °C ein feines Pulver erhalten. Davon wurden 300 g in 1 l Wasser unter gleichzeitiger Zugabe von 54 ml N-NaOH eingetragen und gerührt. Diese NaOH-Menge reicht aus, um einen Anteil von 1,55 Gew.-% des Polymerisats in die Na-Salzform überzuführen. Nach 5 min war eine latexartige Dispersion entstanden. Der Grindometertest liess keine Partikel über 10 μm erkennen. Weniger als 20% der Teilchen lagen im Grössenbereich von 5 bis 10 μm. Die Dispersion wurde wie in Beispiel 1 zu Tablettenüberzügen verarbeitet, wobei ein glatter, in künstlichem Magensaft resistenter Überzug entstand. In künstlichem Darmsaft von pH 7,5 zerfallen die Tabletten innerhalb 15 min.

Beispiel 3

In 220 g Wasser werden 100 g eines gefriergetrockneten Emulsionspolymerisats aus gleichen Gewichtsteilen Methacrylsäure und Acrylsäureäthylester eingetragen und unter Rühren tropfenweise mit 3 g einer wässrigen, 1-normalen Ammoniaklösung versetzt. Diese $NH_3$-Menge reicht aus, um einen Anteil von 0,26 Gew.-% des Polymerisats in die Ammoniumsalzform überzuführen. Nach 15 min Rühren ist eine latexartige Dispersion entstanden. Mehr als 99,5% des Materials ist redispergiert und passiert ein Sieb von 0,1 mm lichter Maschenweite. Der Grindometertest lässt keine Partikel über 5 μm erkennen. Die Brookfield-Viskosität beträgt 12,5 mPa.s. Die Dispersion wurde wie in Beispiel 1 zu Tablettenüberzügen verarbeitet, wobei ein Überzug mit den gleichen Eigenschaften wie in Beispiel 1 erhalten wurde.

Beispiel 4

300 g eines gefriergetrockneten Emulsionspolymerisats aus 50 Gew.-% Dimethylamino-neopentyl-methacrylat, 30 Gew.-% Äthylacrylat und 20 Gew.-% Methylmethacrylat wurden zu 300 g Wasser gegeben und kontinuierlich durch einen Homogenisator (Fryma-Mill) gepumpt. Dabei wurden 42 g einer 1-molaren wässrigen Salzsäurelösung innerhalb 10 min zugegeben, wodurch ein Anteil von 2,8 Gew.-% des Polymerisats in die Hydrochlorid-Salzform übergeführt wurde. Durch weiteres Homogenisieren während 20 min entstand eine feine milchige Dispersion, die wie im Beispiel 1 zum Überziehen von Arzneiformen eingesetzt wurde und einen in künstlichem Magensaft löslichen Überzug ergab.

Beispiel 5

Eine Dispersion mit einem Feststoffgehalt von 30 Gew.-% wurde durch Emulsionspolymerisation von 150 g Methacrylsäure und 150 g Äthylacrylat in 700 g Wasser hergestellt. Nach Abschluss der Polymerisation wurde eine Lösung von 3,6 g NaOH in 100 g Wasser zugesetzt, wodurch ein Anteil von 2,6 Gew.-% des Polymerisats in die Na-Salzform übergeführt wurde. Die Mischung wurde 30 min gerührt und danach gefriergetrocknet. Der zurückbleibende Polymerisatkuchen wurde gemahlen und in einer Lösung von 30 g eines nichtionischen Emulgiermittels («Tween 80»®) in 1 kg Wasser durch intensives Rühren mit einem Schnellrührer dispergiert. Zu der entstandenen Dispersion wurden 75 g Polyäthylenglykol (Molekulargewicht 6000) in 150 g Wasser und 100 g Talkum zugesetzt und die erhaltene Beschichtungsmischung als Isolierschicht auf 10 kg Tabletten in üblicher Weise aufgesprüht.

### Beispiel 6

250 g eines gefriergetrockneten Pulvers aus einem Emulsionspolymerisat aus 125 g Methacrylsäure und 125 g Äthylacrylat wurden mit 4,8 g trokkenem Tris(hydroxymethyl)-aminomethan gemischt, wodurch ein Anteil von 1,4 Gew.-% des Polymerisats in die entsprechende Salzform überführbar ist. Die Mischung wurde unter Rühren mit einem Schnellrührer in Anteilen von je 20 g zu 750 g Wasser gegeben.

Nachdem das ganze Pulver zugegeben war, wurde noch 15 min weitergerührt und die Dispersion anschliessend als Überzugsdispersion verwendet.

### Beispiel 7

Das Verfahren gemäss Beispiel 1 wurde wiederholt, jedoch wurde eine Dispersion aus 50 Gew.-% Methacrylsäure, 45 Gew.-% Äthylacrylat und 5 Gew.-% N-Vinylpyrrolidon eingesetzt. Der damit hergestellte Überzug hatte ähnliche Löslichkeitseigenschaften wie derjenige nach Beispiel 1.

### Beispiel 8

Das Verfahren gemäss Beispiel 2 wurde wiederholt, jedoch wurde eine Dispersion aus 30 Gew.-% Methacrylsäure, 60 Gew.-% Methylmethacrylat und 10 Gew.-% Styrol verwendet. Der damit hergestellte Überzug hatte ähnliche Löslichkeitseigenschaften wie derjenige nach Beispiel 2.

### Patentansprüche

1. Verfahren zum Überziehen von Arzneiformen mittels eines wässrigen Überzugsmittels, welches als Bindemittel einen Latex eines Emulsionspolymerisats aus

A) 20 bis 85 Gew.-% Einheiten wenigstens eines Alkylesters der Acryl- und/oder Methacrylsäure,

B) 80 bis 15 Gew.-% Einheiten wenigstens eines zur Salzbildung befähigten, radikalisch polymerisierbaren Monomeren

C) gegebenenfalls bis zu 30 Gew.-% Einheiten von einem oder mehreren anderen radikalisch polymerisierbaren Monomeren enthält, durch Auftragen des wässrigen Überzugsmittels auf die Arzneiformen und Verdunsten des Wassers unter Bedingungen, unter denen die Latexteilchen verfilmen, dadurch gekennzeichnet, dass man das Emulsionspolymerisat, worin bis zu 10 Gew.-% (bezogen auf das Emulsionspolymerisat) der Monomereinheiten B in der Salzform vorliegen können, in Form eines trockenen Pulvers, dessen Körnchen aus lose aggregierten Feinpartikeln aufgebaut sind, in Wasser einträgt und in Gegenwart einer Menge eines zur Salzbildung mit den Monomereinheiten B befähigten Mittels, die so bemessen ist, dass nach ihrer Umsetzung mit den Monomereinheiten B insgesamt 0,1 bis 10, vorzugsweise 0,5 bis 5 Gew.-% dieser Einheiten, bezogen auf das Gewicht des Emulsionspolymerisats, in Salzform vorliegen, zu wenigstens 80 Gew.-% zu Latexteilchen redispergiert.

2. Pulverförmiges Gemisch zur Herstellung eines wässrigen Überzugsmittels für Arzneiformen, enthaltend

1) ein Emulsionspolymerisat aus

A) 20 bis 85 Gew.-% Einheiten wenigstens eines Alkylesters der Acryl- und/oder Methacrylsäure,

B) 80 bis 15 Gew.-% Einheiten wenigstens eines zur Salzbildung befähigten, radikalisch polymerisierbaren Monomeren

C) gegebenenfalls bis zu 30 Gew.-% Einheiten von einem oder mehreren anderen radikalisch polymerisierbaren Monomeren, dessen Körnchen aus lose aggregierten Feinpartikeln aufgebaut sind, und

2) eine Menge eines pulverförmigen, zur Salzbildung mit den Monomereinheiten B befähigten Mittels, die so bemessen ist, dass nach ihrer Umsetzung mit den Monomereinheiten B insgesamt 0,1 bis 10, vorzugsweise 0,5 bis 5 Gew.% dieser Einheiten, bezogen auf das Gewicht des Emulsionspolymerisats in der Salzform vorliegen.

### Claims

1. Method of coating pharmaceutical preparations by means of an aqueous coating agent which contains as binder a latex of an emulsions polymer consisting of

A) 20 to 85% by weight of at least one alkyl ester of acrylic and/or methacrylic acid,

B) 80 to 15% by weight of units of at least one radically polymerisable monomer capable of salt formation

C) optionally up to 30% by weight of units of one or more other radically polymerisable monomer, by applying the aqueous coating agent to the pharmaceutical preparation and evaporating the water under conditions under which the latex particles form a film, characterised in that the emulsion polymer, in which up to 10% by weight (based on the emulsion polymer) of the monomer units B may be present in salt form, is added to water in the form of a dry powder the particles of which are formed from loosely aggregated fine particles and the emulsion polymer is redispersed, in a quantity of at least 80% by weight, to form latex particles, in the presence of a quantity of an agent capable of salt formation with the monomer units B, the quantity being such that after its reaction with the monomer units B, a total of 0.1 to 10, preferably 0.5 to 5% by weight of these units, based on the weight of the emulsion polymer, are present in salt form.

2. Powdered mixture for preparing an aqueous coating agent for pharmaceutical preparations, containing

1) an emulsion polymer consisting of

A) 20 to 85% by weight of units of at least one alkylester of acrylic and/or methacrylic acid,

B) 80 to 15% by weight of units of at least one radically polymerisable monomer capable of salt formation,

C) optionally up to 30% by weight of units of one or more other radically polymerisable monomers, the particles of which are formed from loosely aggregated fine particles, and

2) a quantity of a powdered agent capable of salt formation with the monomer units B, the quantity being such that after its reaction with the monomer units B a total of 0.1 to 10, preferably 0.5 to 5% by weight of these units, based on the weight of the emulsion polymer, are present in salt form.

**Revendications**

1. Procédé de revêtement de formes médicamenteuses au moyen d'un produit d'enduction aqueux qui contient, en tant que liant, un latex d'un produit de polymérisation en émulsion de

A) 20 à 85% en poids d'unités d'au moins un ester alkylique de l'acide acrylique et/ou méthacrylique,

B) 80 à 15% en poids d'unités d'au moins un monomère susceptible de polymérisation radicalaire et capable de former un sel,

C) le cas échéant, jusqu'à 30% en poids d'unités d'un ou de plusieurs autres monomères susceptibles de polymérisation radicalaire,

par application du produit d'enduction aqueux sur les formes médicamenteuses et vaporisation de l'eau dans des conditions dans lesquelles les particules de latex forment un film, caractérisé en ce que l'on introduit dans l'eau le produit de polymérisation en émulsion, dans lequel jusqu'à 10% en poids (par rapport au produit de polymérisation en émulsion) des unités monomères B peuvent se présenter sous la forme de sel, à l'état d'une poudre sèche dont les grains sont composés de fines particules agrégées sans adhérence, et on le redisperse, pour 80% en poids au moins, en particules de latex en présence d'une quantité d'un agent capable de former un sel avec les unités de monomère B, quantité qui est choisie de telle manière qu'au total, après sa réaction avec les unités de monomère B, 0,1 à 10%, de préférence 0,5 à 5% en poids de ces unités, par rapport au poids du produit de polymérisation en émulsion, se présentent sous la forme de sel.

2. Mélange en poudre pour la préparation d'un produit d'enduction aqueux pour des formes médicamenteuses, contenant

1) un produit de polymérisation en émulsion de

A) 20 à 85% en poids d'unités d'au moins un ester alkylique de l'acide acrylique et/ou méthacrylique,

B) 80 à 15% en poids d'unités d'au moins un monomère susceptible de polymérisation radicalaire et capable de former un sel,

C) le cas échéant, jusqu'à 30% en poids d'unités d'un ou de plusieurs autres monomères susceptibles de polymérisation radicalaire, dont les grains sont composés de fines particules agrégées de façon peu cohérente et

2) une quantité d'un produit en poudre capable de former un sel avec les unités de monomère B, cette quantité étant choisie de telle manière qu'au total, après sa réaction avec les unités de monomère B, 0,1 à 10%, de préférence 0,5 à 5% en poids de ces unités, par rapport au poids du produit de polymérisation en émulsion, se présentent, sous la forme de sel.